# EUROPEAN PATENT APPLICATION

(11) **EP 4 338 711 A1**
(43) Date of publication of application: **20.03.2024**
(21) Application number: 22806474.7
(22) Date of filing: 22.04.2022
(51) Int. Cl.: A61F 2/82

(54) **MEDICAL STENT**

(30) Priority: 12.05.2021 CN 202110514247
(71) Applicant: Shanghai Intervascular MedTech Co., Ltd., Shanghai 201318 (CN)
(72) Inventor: ZHANG, Yu, Shanghai 201318 (CN); CAO, Yang, Shanghai 201318 (CN); ZHU, Qing, Shanghai 201318 (CN); ZHANG, Linlin, Shanghai 201318 (CN)
(74) Representative: Lavoix
(86) International application number: PCT/CN2022/088638
(87) International publication number: WO 2022/237504

(57) **Abstract**

The present invention provides a medical stent. The medical stent includes a covered segment and a naked segment. The covered segment includes a cut stent body and a film material arranged on the cut stent body. At least one end of the covered segment is connected with the naked segment, and at least one of the naked segments includes a braided stent body. The covered segment includes a first section, and the medical stent is configured such that an inner diameter of the first section in a first expansion state is smaller than an inner diameter of the cut stent body in a free state, and when the first section is subjected to a radially outward force, the first section expands radially outward. The medical stent has good flexibility and support, is easy to push, meets the needs of use, and can adjust the inner diameter of the first section according to actual needs, and has good universal applicability.

## Description

### TECHNICAL FIELD

The present invention relates to the field of medical devices and, in particular, to a medical stent.

### BACKGROUND

Due to liver cirrhosis, main portal vein obstruction, hepatic vein obstruction or other unknown reasons, portal vein pressure continues to increase, commonly known as portal hypertension. The blood flow of portal hypertension manifests that the portal blood cannot pass through the liver and flow back into the inferior vena cava smoothly, and at the same time, the communicating branch between the portal and systemic veins is opened. A large amount of portal blood enters the systemic circulation directly through the communicating branch without passing through the liver. Clinical symptoms such as Esophageal vein dilatation, splenomegaly and hypersplenism, liver decompensation and ascites, portal hypertension, gastrointestinal vascular wall may occur, and bleeding from esophageal and gastric varices may also occur in severe cases.

Transjugular intrahepatic portosystemic shunt (TIPS) is a treatment method for portal hypertension developed on the basis of transjugular venography and percutaneous jugular hepatic puncture. The treatment allows the venous and portal vein channels to be supported by implanting stents in the channels, which can maintain patency for a long time. The stents in the prior art have the disadvantages of poor flexibility, difficulty in pushing or poor support when applied to TIPS. In addition, the diameters of existing medical stents are generally fixed in size, respectively 8mm, 10mm, and 12mm. During the operation, the operator chooses a stent with a matching diameter according to the actual needs. When there is no suitable stent, the operator will choose a stent with a larger diameter. This can reduce portal hypertension, but it will also increase the risk of hepatic encephalopathy in the later stage.

### SUMMARY OF THE INVENTION

An object of the present invention is to provide a medical stent, which has good flexibility and support, and meets the requirements of TIPS.

In order to achieve the above object, the present invention provides a medical stent, comprising a covered segment and a naked segment; wherein the covered segment comprises a cut stent body and a film material arranged on the cut stent body; at least one end of the covered segment is connected with a naked segment, and at least one of the naked segments comprises a braided stent body;
wherein the covered segment comprises a first section, and the medical stent is configured such that an inner diameter of the first section in a first expansion state is smaller than an inner diameter of the cut stent body in a free state, and when the first section is subjected to a radially outward force, the first section expands radially outward.

Optionally, the braided stent body is formed by braiding a wire; the medical stent further comprises a first constraint member, and the first constraint member is arranged on the braided stent body and constrains an end of the wire.

Optionally, the braided stent body is in hot-melting connection with the film material; and/or, the medical stent further comprises a connecting tape, and the connecting tape passes through a hole on the braided stent body and is in hot-melting connection with the film material.

Optionally, 3-9 holes are formed on the braided stent body in an axial direction of the medical stent; and/or, 4-8 holes are formed on the braided stent body in a circumferential direction of the medical stent.

Optionally, the medical stent is a self-expanding stent, and when the medical stent is in the first expansion state, a region of the cut stent body corresponding to the first section is subjected to a radial constraint force that is greater than the self-expanding force of the cut stent body in the free state.

Optionally, the film material is at least coated on an outer surface of the cut stent body, and an inner diameter of a region of the film material arranged on the outer surface of the cut stent body corresponding to the first section is smaller than an outer diameter of the cut stent body in the free state when the medical stent is in the first expansion state so that the film material exerts the radial constraint force on the cut stent body; when the first section is subjected to the external force, the region of the film material corresponding to the first section is plastically deformed so that the radial constraint force is reduced and the first section expands radially outward.

Optionally, the medical stent further comprises an annular second constraint piece sleeved over the first section;
wherein the second constraint member is configured such that an inner diameter of the second constraint member in the first expansion state is smaller than an outer diameter of the covered segment in the free state, and the second constraint member exerts the radial constraint force on the cut stent body, and when the first section is subjected to the external force, the second constraint member is plastically deformed or broken and the radial constraint force is reduced, so that the first section is caused to expand radially outward.

Optionally, the second constraint member is formed by connecting both ends of a linear structure; and/or, the second constraint member is provided with a weakened structure to reduce a strength of the second constraint member.

Optionally, the weakened structure comprises at least one of a hole and a groove.

Compared with the prior art, the medical stent of the present invention has the following advantages: first, the aforementioned medical stent includes a covered segment and a naked segment; wherein the covered segment includes a cut stent body and a film material arranged on the cut stent body; at least one end of the covered segment is connected with a naked segment, and at least one of the naked segments comprises a braided stent body; wherein the covered segment includes a first section, and the medical stent is configured such that an inner diameter of the first section in a first expansion state is smaller than an inner diameter of the cut stent body in a free state, and when the first section is subjected to a radially outward force, the first section expands radially outward. The medical stent has both good support and flexibility, and is especially suitable for TIPS. During the practical use of the medical stent, an external force can be applied to the covered segment according to actual needs to change the shape of the first section, in order to expand the applicability of the medical stent. For TIPS, changing the inner diameter of the first section to make the medical stent with a suitable size can effectively reduce the occurrence probability of hepatic encephalopathy.

Further, the naked segment is directly in hot-melting connection with the film material or the naked segment is in hot-melting connection with the film material through a connecting tape, so that the naked segment and the covered segment are firmly connected and the performance of the medical stent is improved.

### BRIEF DESCRIPTION OF THE DRAWINGS

The present invention will be better understood with reference to the accompanying drawings provided without limiting the invention, in which:
Fig. 1 is a schematic structural diagram of a medical stent according to an embodiment of the present invention, in which the braided stent body is in hot-melting connection with the film material.
Fig. 2 is a schematic structural diagram of a medical stent according to an embodiment of the present invention, in which the braided stent body is in hot-melting connection with the film material through a connecting tape.
Fig. 3 is a schematic structural diagram of a medical stent according to an embodiment of the present invention, in which the film material provides a radial constraint force on the cut stent body.
Fig. 4 is a schematic structural diagram of a medical stent according to an embodiment of the present invention, in which the second constraint member provides a radial constraint force on the cut stent body.
Fig. 5 is a schematic structural diagram of a second constraint member in the medical stent shown in Fig. 4.
Fig. 6 is a schematic structural diagram of a second constraint member in the medical stent shown in Fig. 4, in which a weakened structure is formed on the second constraint member.

Reference numerals are listed as follows: 110, covered segment; 110a, first section; 110b, proximal section; 110c, distal section; 111, cut stent body; 112, film material; 121, braided stent body; 130, first constraint member; 140, connecting tape; 150, second constraint member; 151, weakened structure; 160, radiopaque element.

### DETAILED DESCRIPTION

Embodiments of the present invention are described below through specific examples, and those skilled in the art can easily understand other advantages and effects of the present invention from the content disclosed in this specification. The present invention can also be implemented or applied through other different specific implementation modes, and various modifications or changes can be made to the details in this specification based on different viewpoints and applications without departing from the spirit of the present invention. It should be noted that the diagrams provided in this embodiment are only schematically illustrating the basic idea of the present invention, and only the components related to the present invention are shown in the diagrams rather than the number, shape and size of the components in actual implementation. The type, quantity and proportion of each component can be changed arbitrarily during actual implementation, and the component distribution may also be more complicated.

In addition, each embodiment of the content described below has one or more technical features respectively, but this does not mean that the inventor must implement all the technical features in any embodiment at the same time, or can only implement some or all of the technical features in different embodiments separately. In other words, on the premise that the implementation is possible, those skilled in the art can selectively implement some or all of the technical features in any embodiment according to the disclosure of the present invention and depending on design specifications or implementation requirements, or selectively implement a combination of some or all of the technical features in multiple embodiments, thereby increasing the flexibility of the implementation of the present invention.

As used herein, the singular forms of "a", "an", and "the", include plural references, and the plural form "plurality" includes more than two references, unless the context clearly dictates otherwise. As used herein, the term "or" refers to "and/or" unless the context clearly dictates otherwise. In addition, the terms "mounted", "connected", "connected" are to be interpreted broadly. For example, it may be a fixed connection, a detachable connection, or a integral connection; it may be a mechanical connection or an electrical connection; it may be directly connected or indirectly connected through an intermediary, and it may be the internal communication of two elements or the interaction relationship between two elements. Those of ordinary skill in the art can understand the specific meanings of the above terms in the present invention according to specific situations.

To make the objectives, advantages and features of the present invention clearer, the present invention will be further described in detail below with reference to the figures. It is noted that the figures are provided in a very simplified form not necessarily presented to scale, with their only intention to facilitate convenience and clarity in explaining the disclosed embodiments. Throughout the figures, like reference numerals indicate the same or analogous components or elements.

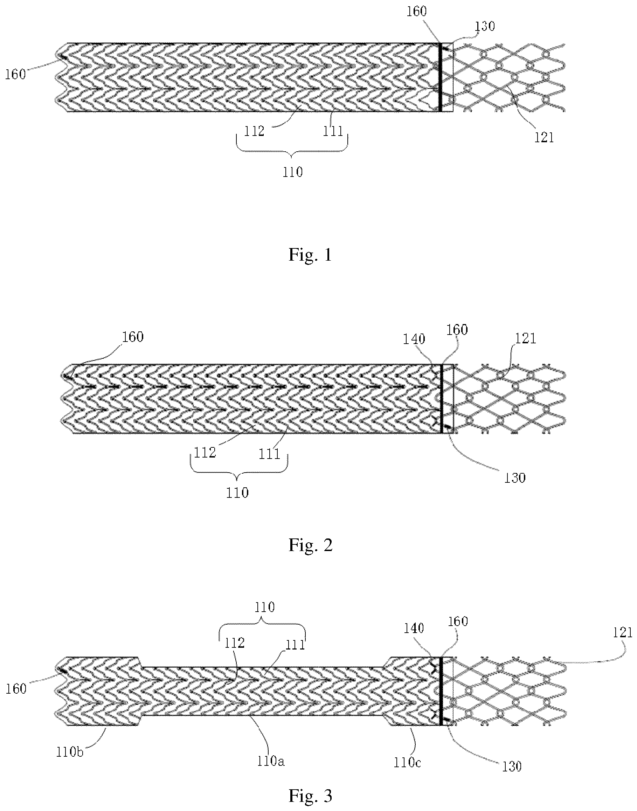

Figs. 1-4 are schematic structural diagrams of a medical stent according to an embodiment of the present invention. Please refer to Figs. 1-4, the medical stent includes a covered segment 110 and a naked segment. The covered segment 110 includes a cut stent body 111 and a film material 112, and the film material 112 is coated on the cut stent body 111. The covered segment 110 includes a first section 110a, and the medical stent is configured such that the inner diameter of the first section 110a in the first expansion state is smaller than the inner diameter of the cut stent body 111 in the free state, and when the first section 110a is subjected to a radially outward force, the first section 110a expands radially outward. At least one end of the covered segment 110 is connected with the naked segment, and the naked segment includes a braided stent body 121. It can be understood that the cut stent body 111 is formed by cutting a tube and then shaped by heat treatment, and the braided stent body 121 is formed by braiding a wire material and then shaped by heat treatment. The medical stent is intended to be implanted at a predetermined location in the patient's body. For example, during transjugular intrahepatic portosystemic shunt (TIPS), the medical stent is intended to be implanted in the liver and used to construct a blood flow channel between the hepatic vein and the portal vein, the naked segment is used to allow part of the blood flow to flow into the liver tissue to prevent the liver tissue from shrinking.

The medical stent according to an embodiment of the present invention combines the cut stent body 111 and the braided stent body 121, which makes it not only has good support performance, so that a stable blood flow channel can be built in the liver, but also has good flexibility, so that the medical stent can be smoothly delivered to the predetermined location. It should be noted that, when the medical stent includes only one naked segment, the naked segment is located at the distal end of the covered segment 110, so that the naked segment will located at the portal vein after the medical stent is implanted in the liver. The "distal end" described here is the relative orientation, relative position, or relative direction for elements or acts from the perspective of the doctor who uses the medical stent. Although it is not limited herein, the "distal end" usually refers to the end that first enters the patient's body, as opposed to the "proximal end" which refers to the end that is closer to the doctor.

The medical stent is preferably a self-expanding stent. In some embodiments, the cut stent body 111 is made from medical low-carbon stainless steel (316L stainless steel), or nickel-titanium alloy or other shape memory alloys. The length of the cut stent body 111 may be 40mm-100mm, preferably 40mm-80mm, more preferably 70mm-80mm. The width of the cut stent rod is 0.09mm-0.25mm, preferably 0.12mm-0.18mm. The thickness of the cut stent body 111 is 0.18mm-0.25mm. The stent rods may be shaped in the form of a sinusoidal or triangular waveform, and each stent rod may include 4-8 peaks, preferably 4-6 peaks, in the circumferential direction of the medical stent. The film material 112 covers at least the outer surface of the cut stent body 111, and preferably the film material 112 covers both the inner surface and the outer surface of the cut stent body 111. The thickness of the film material 112 is 20um-120um, preferably 25um-60um. The film material 112 have good permeability, and may be PET or ePTFE, preferably ePTFE. The diameter of the wire used to braiding the braided stent body 121 is 0.15 mm to 0.25 mm, preferably 0.20 mm to 0.25 mm, and may be made from a shape memory alloy, specifically at least one of Au-Cd, Ag-Cd, Cu-Zn, Cu-Zn-Al, Cu-Zn-Sn, Cu-Zn-Si, Cu-Sn, Cu-Zn-Ga, Au-Cu-Zn, NiAl, Fe-Pt, Ti-Ni, Ti-Ni-Pd , Ti-Nb, U-Nb, Fe-Mn-Si. The length of the braided stent body 121 may be 15mm-45mm, preferably 20mm-40mm. The holes formed on the braided stent body 121 are each shaped as a diamond. Along the axial direction of the medical stent, the number of the diamond-shaped holes is 3-9, preferably 3-8, more preferably 4-8, and along the circumferential direction of the medical stent, the number of the diamond-shaped holes is 4-8, preferably 4-6.

Preferably, the medical stent further includes a first constraint member 130. The first constraint member 130 is arranged on the braided stent body 121, and constrains an end of the wire to structurally maintain the braided stent body 121, thereby avoiding disassembly. In some embodiments, the first constraint member 130 is a tube with an inner diameter slightly larger than the outer diameter of the wire, so that the end of the wire can extend into the inside of the tube and be constrained by the tube. Depending on the diameter of the wire, the inner diameter of the tube may be 0.0075 inches to 0.0100 inches, preferably 0.0075 inches to 0.0095 inches. In addition, the number of the first constraint members 130 is equal to the number of wires used to braiding the braided stent body 121. For example, when the braided stent body 121 is braided from one wire, the number of the constraint member 130 is one; when there are two wires, the number of the first constraint members 130 is two, and each first constraint member 130 constrains two ends.

Optionally, as shown in Fig. 1, the braided stent body 121 is directly connected to the film material 112 of the covered segment 110 by hot-melting; and/or, please refer to Fig. 2, the medical stent further includes a connecting tape 140 which passes through the holes of the braided stent body 121 and is connected with the film material 112 by hot-melting. The material of the connection belt 140 is preferably the same as that of the film material 112, which is beneficial to the integration of the film material 112 with the connecting tape 140, and further improves the connection strength between the covered segment 110 and the naked segment. Generally, the connecting tape 140 is connected to the film material 112 after passing through the holes of the braided stent body 121 closest to the covered segment 110.

In addition, more than two radiopaque elements 160 may be arranged on the covered segment 110. The more than two radiopaque elements 160 may be arranged on opposite ends of the covered segment 110, and at least one of the radiopaque elements 160 is a radiopaque ring. When the medical stent includes only one naked segment, the radiopaque ring is arranged closer to the naked segment. The radiopaque element can be made of radiopaque metals such as platinum-iridium alloy, platinum-tungsten alloy, stainless steel, gold, and tantalum.

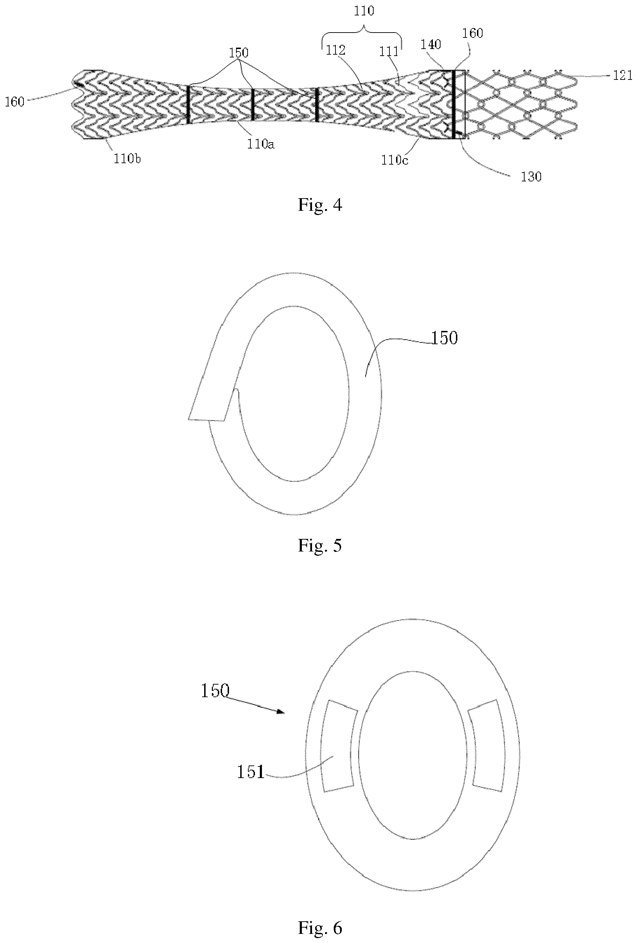

Further, please focus on referring to Figs. 3 and 4, the covered segment 110 includes the first section 110a, and the first section 110a may extend from the proximal end to the distal end of the covered segment 110 (that is, the entire covered segment 110 is completely composed of the first section 110a). Alternatively, the first section 110a may occupy a partial area of the covered segment 110, for example, the first section 110a is located in the middle area of the covered segment 110, so that the covered segment 110 includes a proximal section 110b, the first section 110a and a distal section 110c that are sequentially connected along an axial direction (as shown in Fig. 3).

When the medical stent is in the first expansion state, the first section 110a has a first inner diameter d₁. When the cut stent body 111 is in a free state, the cut stent body 111 has a second inner diameter D. The first inner diameter d₁ is smaller than the second inner diameter D. When the medical stent is in the first expansion state, and when the first section 110a is subjected to a radially outward force, the first section 110a can expand radially outward. In other words, the first section 110a has a changable inner diameter. In practice, when the medical stent is delivered to a predetermined location in human body and then released, it can expand to the first expansion state due to self-expansion, and then continue to enlarge the inner diameter of the first section 110a if an external force is applied to the first section 110a to further expand the first section 110a.

Therefore, the medical stent is especially suitable for TIPS. Specifically, when the patient actually needs a medical stent with an inner diameter of d₂, the operator can use the medical stent according to this embodiment as long as it satisfies d₁≤d₂ ≤D. When the size of the medical stent satisfies d₁<d₂<D, the operator can release the medical stent and expand the medical stent to the first expansion state, and then use a balloon to apply an external force to the first section 110a to continue expanding the first section 110a until the diameter of the first section 110a reaches d₂. Therefore, the medical stent has good universal applicability. When the medical stent is applied to TIPS, it can not only reduce portal hypertension, but also greatly reduce the occurrence probability of hepatic encephalopathy. In addition, in the case that the first section 110a is expanded but not reaches the second inner diameter D, the first section 110a can be re-expanded by a balloon to achieve a larger shunt once in-stent restenosis occurs at a later stage.

For the self-expanding stent, the "free state" refers to the state when the cut stent body 111 is not subjected to radial constraint force from the outside. Thus, in the embodiment of the present invention, the region of the cut stent body 111 corresponding to the first section 110a can be subjected to a radial constraint force greater than the self-expanding force of the cut stent body 111 in a free state so that the medical stent is switch to the first expansion state. The self-expanding force is a radially outward elastic force generated by the cut stent body 111 when it is subjected to a radial constraint force. The cut stent body 111 generates different self-expanding forces according to the difference in the inner diameter of the region corresponding to the first section 110a. Optionally, the smaller the inner diameter of the cut stent body 111 at a region thereof corresponding to the first section 110a, the greater the self-expanding force. Optionally, when the stent is in the free state, no self-expanding force is generated.

Specifically, in an optional implementation, please focus on referring to Fig. 3, the medical stent is configured so that the inner diameter of the film material 112 on the surface of the first section 110a is smaller than the outer diameter of the cut stent body 111 in the free state when the medical stent is in the first expansion state, and thus the region of the film material 112 corresponding to the first section 110a applies the radial constraint force to the cut stent body 111 and prevents the cut stent body 111 from further expansion. As a result, the first section 110a has the first inner diameter d₁. When the first section 110a is subjected to the external force, the region of the film material 112 corresponding to the first section 110a is plastically deformed and the radial constraint force is reduced to be smaller than the self-expanding force of the cut stent body 111, so that the cut stent body 111 can be further expanded. The "plastic deformation" refers to a deformation that cannot be recovered by itself. That is to say, when the external force is cancelled, the current inner diameter of the first section 110a is larger than the first inner diameter d₁ in the first expansion state.

Please refer to Fig. 4 again, in an alternative implementation, the medical stent includes an annular second constraint member 150 sleeved over the outer surface of the first section 110a. The second constraint member 150 is configured such that the inner diameter of the second constraint member 150 is smaller than the outer diameter of the cut stent body 111 when it is in the free state, and thus the second constraint member 150 exerts the radial constraint force on the first section 110a and prevents the cut stent body 111 from further expansion. When the first section 110a is subjected to the external force, the second constraint member 150 plasticly deforms and reduces the radial constraint force to be smaller than the self-expanding force of the cut stent body 111, so that the the cut stent body 111 can be further expanded to cause its first section 110a to further expand accordingly. It can be understood that when the external force increases to a predetermined value, the second constraint member 150 may break. The predetermined value is determined according to the strength of the second constraint member 150 itself.

Please refer to Fig. 5, optionally, the number of the second constraint member 150 is at least one, and each second constraint member may be formed by connecting both ends of a linear structure. When the external force reaches the predetermined value, the second constraint member 150 breaks at the connection between the ends. The predetermined value is determined according to the connection strength of the connection. Alternatively, please refer to Fig. 6, the second constraint member 150 is provided with a weakened structure 151. The weakened structure 151 reduces the strength of the second constraint member 150, so that it is easy to be plasticly deformed or broken when it is subjected to the external force. It can be understood that, in this case, the second constraint member 150 has a certain width to allow the weakened structure 151 to be disposed. In this embodiment, the weakened structure is, for example, a hole, and the number and shape of the holes are not particularly limited in this embodiment of the present invention. Generally, the number of the holes can be 4 to 50, preferably 8 to 20, and each hole may have an area of 1mm²~4mm², preferably 3mm²~4mm².

It should be noted that, when the medical stent is applied to TIPS, the medical stent is implanted in the liver, and the liver tissue is attached to the outer surface of the medical stent. When the second constraint member 150 breaks, the second constraint member 150 does not break away from the covered segment 110 under the pressure of the liver tissue.

The using process will be introduced by taking the medical stent including the first section 110a and applying the medical stent to TIPS as an example. In this process, for example, the inner diameter d₂ of the medical stent, the first inner diameter d₁ of the first section 110a, and the second inner diameter D of the cut stent body 111 satisfy d₁<d₂<D.

First, the operator prepares the shunt and performs a preoperative evaluation.

Next, the operator calculates the value of PPG (portal pressure gradient) according to the preoperative evaluation, and selects a medical stent with an appropriate inner diameter.

Next, the medical stent is implanted into the liver, and the medical stent is self-expanded to the first expansion state.

Then, a balloon introduced to apply an external force to the first section 110a of the medical stent, so that the first section 110a is further expanded until the inner diameter of the first section 110a reaches d₂.

At a later stage, if restenosis occurs in the medical stent, the operator can re-introduce the balloon and expand the first section 110a again to obtain a larger shunt.

Although the present invention is disclosed above, it is not limited thereto. Those skilled in the art can make various modifications and variations to the present invention without departing from the spirit and scope thereof. Accordingly, the invention is intended to embrace all such modifications and variations if they fall within the scope of the appended claims and equivalents thereof.

## Claims

1. A medical stent, comprising a covered segment and a naked segment; wherein the covered segment comprises a cut stent body and a film material arranged on the cut stent body; at least one end of the covered segment is connected with a naked segment, and at least one of the naked segments comprises a braided stent body;
wherein the covered segment comprises a first section, and the medical stent is configured such that an inner diameter of the first section in a first expansion state is smaller than an inner diameter of the cut stent body in a free state, and when the first section is subjected to a radially outward force, the first section expands radially outward.

2. The medical stent according to claim 1, wherein the braided stent body is formed by braiding a wire; the medical stent further comprises a first constraint member, and the first constraint member is arranged on the braided stent body and constrains an end of the wire.

3. The medical stent according to claim 1, wherein the braided stent body is in hot-melting connection with the film material; and/or, the medical stent further comprises a connecting tape, and the connecting tape passes through a hole on the braided stent body and is in hot-melting connection with the film material.

4. The medical stent according to any one of claims 1-3, wherein 3-9 holes are formed on the braided stent body in an axial direction of the medical stent; and/or, 4-8 holes are formed on the braided stent body in a circumferential direction of the medical stent.

5. The medical stent according to claim 1, wherein the medical stent is a self-expanding stent, and when the medical stent is in the first expansion state, a region of the cut stent body corresponding to the first section is subjected to a radial constraint force that is greater than the self-expanding force of the cut stent body in the free state.

6. The medical stent according to claim 5, wherein the film material is at least coated on an outer surface of the cut stent body, and an inner diameter of a region of the film material arranged on the outer surface of the cut stent body corresponding to the first section is smaller than an outer diameter of the cut stent body in the free state when the medical stent is in the first expansion state so that the film material exerts the radial constraint force on the cut stent body; when the first section is subjected to the external force, the region of the film material corresponding to the first section is plastically deformed so that the radial constraint force is reduced and the first section expands radially outward.

7. The medical stent according to claim 5, further comprising an annular second constraint piece sleeved over the first section;
wherein the second constraint member is configured such that an inner diameter of the second constraint member in the first expansion state is smaller than an outer diameter of the covered segment in the free state, and the second constraint member exerts the radial constraint force on the cut stent body, and when the first section is subjected to the external force, the second constraint member is plastically deformed or broken and the radial constraint force is reduced, so that the first section is caused to expand radially outward.

8. The medical stent according to claim 7, wherein the second constraint member is formed by connecting both ends of a linear structure; and/or, the second constraint member is provided with a weakened structure to reduce a strength of the second constraint member.

9. The medical stent according to claim 8, wherein the weakened structure comprises at least one of a hole and a groove.
